# EUROPÄISCHE PATENTANMELDUNG

(11) **EP 1 053 753 A1**
(43) Veröffentlichungstag der Anmeldung: **22.11.2000**
(21) Anmeldenummer: 99109830.2
(22) Anmeldetag: 19.05.1999
(51) Int. Cl.: A61K 47/42

(54) **Lokaler Wirkstoffträger**

(71) Anmelder: Resorba Chirurgisches Nahtmaterial Franz Hiltner GmbH & Co., 90443 Nürnberg (DE)
(72) Erfinder: Hiltner, Claus Martin, 90408 Nürnberg (DE)
(74) Vertreter: Schneck, Herbert, Dipl.-Phys., Dr.

(57) **Zusammenfassung**

Bei einem lokalen pharmazeutischen Wirkstoffträger auf Kollagenbasis oder anderer resorbierbarer, biokompatibler Stoffe, ist vorgesehen, daß der Wirkstoff in Mikrokapseln eingelagert ist, welche in dem Wirkstoffträger inkooperiert sind.

## Beschreibung

Die Erfindung richtet sich auf einen lokalen pharmazeutischen Wirkstoffträger, insbesondere auf Kollagenbasis.

In den letzten beiden Jahrzehnten wurden verschiedene Entwicklungen vorangetrieben, um im Bereich der operativen Infektionssanierung den chirurgischen Eingriff durch eine lokale antibiotische Therapie zu unterstützen. Der Vorteil der gezielten lokalen Wirkstoffapplikationen liegt in der Möglichkeit, einerseits am Infektionsherd hohe Arzneistoffwirksamkeit zu erzielen, andererseits die oft unerwünschten Nebenwirkungen durch Aufnahme in den Blutkreislauf gering zu halten. Die Anwendung solcher Therapien hat sich besonders in der Knochenchirurgie bei der Behandlung von Knocheninfektionen, wie der Osteomyelitis und der Osteitis, zu einem akzeptierten Standard entwickelt, was nicht zuletzt auf der Entwicklung geeigneter Wirkstoffträger zur lokalen Applikation beruht.

Beispiele sind Knochenzemente mit antiobiotischem Zusatz im Umfeld der Endoprothetik, wirkstofftragende Kugelketten, ein Verbund von Fibrin-Antibiotikum und eine Kombination von resorbierbaren Kollagengelen oder -schwämmen mit antibiotischen Substanzen, wie Taurolidin oder Gentamincin.

In den letzten Jahren haben sich aus den verschiedenen Entwicklungen die gentamicinhaltigen PMMA-Ketten sowie die mit dem gleichen Wirkstoff versehenen resorbierbaren Kollagenschwämme als klinisch geeignet herauskristallisiert. Beiden System gemeinsam ist die für die lokale Infektbekämpfung notwendige Erreichung einer hohen Wirkstoffdosis bereits kurzzeitig nach der Implantation. Die unerwünschten ortho- und nephrotoxischen Nebenwirkungen von Gentamicin werden dabei trotz hoher lokaler Wirkstoffspiegel durch die konzentrierte Arzneimittelfreisetzung durch die zeitversetzte Aufnahme in den Blutkreislauf nicht erreicht. Die lokalen Gentamicinspiegel erreichen dabei eine bis zu 1000-fach höheren Wert wie durch systemische Gabe.

So sehr die hohe lokale Wirkstoffdosis zur Therapie von verschiedenen Infektionen erwünscht ist, sind die bisher bekannten Trägersysteme doch auch mit Nachteilen behaftet.

So liegt ein wesentlicher Nachteil darin, daß der Wirkstoff in den ersten zwei bis vier Tagen weitgehend freigesetzt wird, so daß in der Folgezeit eine effektive lokale Antibiose zurückgeht. Dies beruht einerseits auf der vollständige passiven und aktiven Freisetzung aus dem Kollagenträger und andererseits auf der vollständigen Freigabe des Gentamicins von den oberflächennahen Schichten der PMMA-Kugeln. Ein weiterer Nachteil liegt darin, daß der PMMA-Wirkstoffträger im Gegensatz zu Kollagen, das innerhalb von rund acht Wochen vollständig vom Gewebe um- und abgebaut wird, nicht resorbierbar ist und nach einer gewissen Zeit wird explantiert werden muß.

Bei chronischen Infektionen, wie der häufig in dieser Form anzutreffenden Osteomyelitis, wäre jedoch eine zeitliche Verlängerung einer relativ hohen Wirkstoffkonzentration wünschenswert, da sich die Infektionsherde oft in schwer zugänglichen, häufig schlecht durchbluteten Gewebebereichen befinden und daher auch der lokale Antibiotikatransport behindert sein kann.

Hiervon ausgehend liegt der Erfindung die Aufgabe zugrunde, einen lokalen Wirkstoffträger auf Kollagenbasis so auszugestalten, daß einerseits die Vorteile eines resorbierbaren Trägers voll erhalten bleiben und andererseits eine langfristige, gezielte Wirkstoff-Freisetzung ermöglicht wird.

Diese Aufgabe wird erfindungsgemäß dadurch gelöst, daß der Wirkstoff in Mikrokapseln eingelagert ist, welche in dem Kollagenwirkstoffträger inkooperiert sind.

Vorzugsweise ist vorgesehen, daß der Durchmesser der Wirkstoffkapseln kleiner als 100 µm ist und in Abhängigkeit von dem Anwendungsgebiet eine definierte Wandstärke zur Kontrolle der Freisetzungsdynamik aufweist.

Hierdurch wird ein ganz wesentlicher Vorteil dahingehend erzielt, daß der Wirkstoff zeitverzögert bzw. zeitlich definiert auch über längere Zeiträume freigesetzt werden kann und zur Erzielung der protrahierten Freisetzung der Wirkstoff chemisch nicht verändert werden muß, so daß seine Wirksamkeit und Biokompatibilität unbeeinflußt bleiben und dementsprechend kein gesondertes Zulassungsverfahren erforderlich ist.

In weiterer Ausgestaltung der Erfindung kann vorgesehen sein, daß ein verkapselter Monowirkstoff eingesetzt wird, daß mehrere verschiedene Wirkstoffe in verkapselter Form Verwendung finden oder daß die Wirkstoffdarreichung mit unterschiedlichen Freisetzungsreaktionen erfolgt, z.B. teilweise in nicht und teilweise in verkapselter Form oder in verkapselter Form mit unterschiedlichen Hüllmaterialien und/oder Wandstärken.

Als bioabsorbierbare Substanzen für die Verkapselung kommen Polymeren organischer Säuren, z.B. PGA, PLCA, sowie deren Copolymeren, daneben auch Proteine und Polysacharide vorzugsweise in Betracht.

Ein Verfahren zur Herstellung eines erfindungsgemäßen Wirkstoffträgers mit mikroverkapselten Wirkstoffen sieht vor, daß eine kollagenhaltige, wäßrige Lösung hergestellt wird, in welche die eine pulverförmige oder granulatförmige Konsistenz aufweisenden Mikrokapseln mit dem jeweiligen Wirkstoff gleichmäßig verteilt eingerührt werden, wobei dann eine Trocknung, vorzugsweise Gefriertrocknung erfolgt.

Hierdurch wird, je nach Verfahrensführung ein Lyophilisat, ein Schwamm, eine Folie oder ein Gel aus Kollagen hergestellt, in welchem die Wirkstoffträger in Form von Mikrokapseln äußerst gleichmäßig verteilt und inkooperiert sind.

Das Kollagen kann auch mit Hilfsstoffen geringer pharmakologischer Wirkung beaufschlagt werden, wie beispielsweise mehrwertige Alkohole, z.B. Glyzerin, Propandiol und dergleichen, mit organischen Säuren, mit gelbildenden Substanzen, wie zum Beispiel Hyaluronsäure, Carboxymethylzellulose und dergleichen, und mit Tensiden.

Nachfolgend wird die Erfindung anhand von zwei Ausführungsbeispielen näher erläutert:

### Ausführungsbeispiel 1:

Herstellung einer Augmentationshilfe zur Regeneration von Organgeweben.

In einer Defekthöhle wird ein Kollagenimplantat zur temporaren Defektfüllung mit folgender Zusammensetzung eingebracht:

Der Kollagenimplantatkörper ist aus lyophilisiertem Kollagen tierischen Ursprungs hergestellt. In das Implantat eingebettet sind zwei Sorten von Mikrokapseln. Eine Kapselsorte besteht aus PGA (Polyglykolsäure) mit einem Kapseldurchmesser von 50 µm, die andere Sorte besteht aus Polysacchariden mit einem Kapseldurchmesser von 100 µm.

In die Mikrokapseln sind verschiedene Wachstumsfaktoren mit folgendem Ziel eingearbeitet:

Es soll in einem Zeitraum von ca. zwei Wochen ein Faktor zur Förderung des Fibroblastenwachstums in hoher Konzentration freigesetzt werden. Nach diesem Zeitraum soll ein weiterer Wachstumsfaktor das Einsprießen von Gefäßen (Mikrovaskularisation) in das Wundgewebe nachhaltig über einen Zeitraum von mehreren Wochen unterstützen.

Zum Schutz des Implantats vor bakterieller Kontamination während der Implantation ist daneben eine Dosis von 100 mg Gentamicin in den Kollagenschwamm eingearbeitet, so daß der therapeutische Verlauf des Regenerationsvorganges sich wie folgt darstellen kann:
1. Implantation
2. Antibiotischer Schutz
3. Wachstumsförderung für das Einwandern von Fibroblasten
4. Wachstumsförderung für das Einsprießen von Mikrogefäßen in das Wundgewebe
5. Gewebeaufbau mit zunehmender Resorption des Kollagenimplantats

### Ausführungsbeispiel 2:

Behandlung einer Knochenzyste im Kieferknochen:

Eingesetzt wird ein Implantat aus Kollagen mit einem antibiotischen Schutz durch Vancomicin 200 mg. Nach Herdsanierung wird das Implantat in die Knochenhöhle eingesetzt und mit dem Zahnfleisch abgedeckt und verschlossen.

In dem Kollagenimplantat sind in Mikrokapseln wachstumsfördernde Faktoren zur Knochenbildung und Revaskularisierung eingebettet, die einem bestimmten Freisetzungsprofil folgend in den darauffolgenden sechs bis acht Wochen freigesetzt werden sollen und lokal die Geweberegeneration fördern sollen.

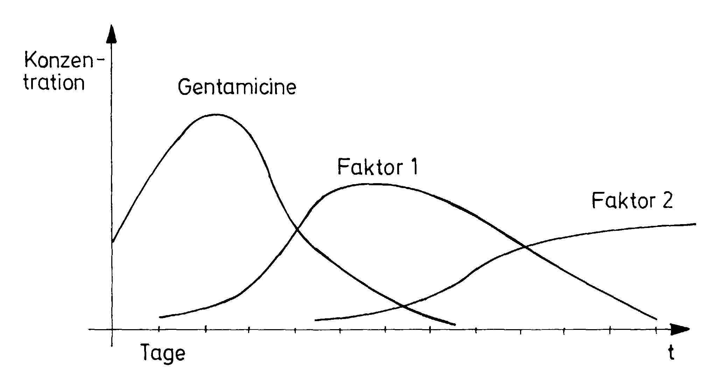

In der beigefügten Zeichnung ist schematisch in einem Konzentrations-Zeit-Diagramm dargestellt, wie es aufgrund der erfindungsgemäßen Ausgestaltung möglich ist, einerseits Gentamicin und andererseits zwei unterschiedliche Faktoren zeitlich gegeneinander versetzt freizusetzen.

## Patentansprüche

1. Lokaler pharmazeutischer Wirkstoffträger auf Kollagenbasis oder anderer resorbierbarer, biokompatibler Stoffe, **dadurch gekennzeichnet, daß** der Wirkstoff in Mikrokapseln eingelagert ist, welche in dem Wirkstoffträger inkooperiert sind.

2. Wirkstoffträger nach Anspruch 1, **dadurch gekennzeichnet, daß** der Durchmesser der Wirkstoffkapseln kleiner als 10 µm ist.

3. Wirkstoffträger nach Anspruch 1, **dadurch gekennzeichnet, daß** in dem Wirkstoffträger ein Monowirkstoff enthalten ist.

4. Wirkstoffträger nach Anspruch 1, **dadurch gekennzeichnet, daß** in den Kapseln unterschiedliche Wirkstoffe enthalten sind.

5. Wirkstoffträger nach Anspruch 1, **dadurch gekennzeichnet, daß** die Wirkstoffkapseln zur Erzielung einer unterschiedlichen Freisetzungsreaktion unterschiedliche Hüllmaterialien und/oder Wandstärken aufweisen.

6. Wirkstoffträger nach Anspruch 1, **dadurch gekennzeichnet, daß** die Wirkstoffkapseln Umhüllungen aus bioabsorbierbaren Substanzen, wie Polymere organischer Säuren, deren Copolymeren und Proteine aufweisen.

7. Wirkstoffträger nach Anspruch 1, **dadurch gekennzeichnet, daß** dem Träger Hilfsstoffe geringer pharmakologischer Wirkung, wie mehrwertige Alkohole, organische Säuren oder gelbildende Substanzen zugesetzt sind.

8. Verfahren zur Herstellung eines Wirkstoffträgers nach einem der Ansprüche 1 bis 7, **dadurch gekennzeichnet, daß** in eine wäßrige Kollagenlösung Mikrokapseln mit Wirkstoff bzw. Wirkstoffen gleichmäßig eingerührt werden und die so gebildete Suspension dann gefriergetrocknet wird.
